# EUROPEAN PATENT APPLICATION

(11) **EP 3 064 200 A1**
(43) Date of publication of application: **07.09.2016**
(21) Application number: 16157531.1
(22) Date of filing: 26.02.2016
(51) Int. Cl.: A61K 31/122, A61K 31/366, A61K 31/4375, A61K 31/7048, A61K 36/062, A61K 36/29, A61K 36/63, A61P 3/06

(54) **COMPOSITION COMPRISING NATURAL SUBSTANCES AND/OR EXTRACTS**

(30) Priority: 03.03.2015 IT UB20150541
(71) Applicant: AZIENDE CHIMICHE RIUNITE ANGELINI FRANCESCO - A.C.R.A.F. - S.p.A., 00181 Roma (IT)
(72) Inventor: FOCANTI, Francesca, 00141 ROMA (IT); RAGNI, Lorella, 60033 CHIARAVALLE (AN) (IT); DONELLI, Daniela, 60131 ANCONA (IT); MILANESE, Claudio, 00143 ROMA (IT)
(74) Representative: Allaix, Roberto

(57) **Abstract**

The present invention relates to a composition comprising a mixture of (i) berberine, or an extract or product containing berberine, (ii) monacolin K, or an extract or product containing monacolins, and (iii) oleuropein, or an extract or product containing oleuropein. Optionally, the composition of the present invention also comprises an antioxidant, in particular ubidecarenone.

## Description

### FIELD OF THE INVENTION

The present invention relates to a composition comprising natural substances and/or extracts. In particular, the present invention relates to a composition comprising berberine, monacolins and oleuropein, or natural extracts and/or products thereof, with a hypercholesterolaemiant and hypertriglyceridaemiant effect.

### PRIOR ART

In Italy, according to recent studies which measured cardiovascular risk factors in adult population samples (men and women between 35 and 74 years old), 21% of men and 23% of women are hypercholesterolaemic (i.e. they have a total cholesterolaemia value greater than or equal to 240 mg/dl, or are under specific treatment), while 37% of men and 34% of women have a condition defined as at-risk (total cholesterolaemia of between 200 and 239 mg/dl).

The situation is significantly worse in the elderly population (men and women between 65 and 74 years old), where, according to the same studies, 24% of men and 39% of women are hypercholesterolaemic, and 36% of men and 38% of women are at risk.

Besides the elderly, women in menopause (average age of 62) constitute a category that is particularly at risk of hypercholesterolaemia. Specifically, in Italy, 36% of women in menopause have a total cholesterolaemia value of greater than or equal to 240 mg/dl, or are under a specific pharmacological treatment, while 38% are in an at-risk condition.

Various risk factors that may contribute to hypercholesterolaemia exist, among which are diet, excess weight and obesity, lack of physical activity, but also the conjunction of metabolic diseases such as diabetes. Also, smoking may damage the blood vessels and accelerate the process of hardening of the arteries.

Cholesterol, together with triglycerides, is the product of conversion of dietary fats which takes place in the liver. Cholesterol and triglycerides are then incorporated into structures known as lipoproteins, to be distributed to the adipose cells via the blood stream.

According to their density, lipoproteins are distinguished as very low density lipoprotein (VLDL), low density lipoprotein (LDL) and high density lipoprotein (HDL).

LDL lipoproteins transport cholesterol synthesized in the liver to the cells of the body, HDL lipoproteins remove excess cholesterol from the various tissues and transport it once again to the liver, which then effects its elimination.

The process of atherosclerosis arises when special cells take up LDL and deposit the fat contained on the walls of blood vessels.

The cholesterol thus deposited causes narrowing of the vessels and may thus lead to heart attacks and strokes. This is why LDL cholesterol is known as bad cholesterol. HDL cholesterol, on the other hand, is considered as good cholesterol, since it is responsible for the removal of the excess cholesterol that is taken to the liver.

The desirable values of LDL cholesterol are up to 100 mg/dl, those of HDL cholesterol are not less than 50 mg/dl, with a total cholesterol value of not more than 200 mg/dl.

Various medicaments and/or food supplements currently exist that are capable of developing positive health effects that can significantly reduce the levels of cholesterol, above all when combined with a controlled diet and healthy physical activity.

The medicaments that are currently marketed comprise, as active principles, statins (mevastatin, lovastatin, simvastatin, pravastatin, fluvastatin, cerivastatin, atorvastatin, rosuvastatin), fibrates (clofibrate, fenofibrate, benzafibrate, ciprofibrate, genfibrozil), nicotinic acid and derivatives thereof (oxyniacic acid, homonicotinic acid, nicotinyl alcohol, nicametate, cyclonicate), and cholesterol absorption inhibitors (ezetimibe).

The food supplements currently marketed comprise various ingredients that are considered to be capable of reducing cholesterol, for instance γ-oryzanol (a component of rice oil), phytosterols or phytostanols (substances contained in products of plant origin containing fats such as dry fruit, vegetable oils and certain vegetables), berberine (quaternary isoquinolene alkaloid present in various plants of the *Berberidacea* family), policosanols (mixtures of aliphatic alcohols derived from sugar cane), monacolins (substances obtained from the fermentation of common cooking rice by a particular yeast, *Monascus purpureus*).

The international patent application published under the number WO 2006/029577 describes a composition for preventing and treating hyperlipidaemia, hypercholesterolaemia and/or cardiovascular disorders, comprising berberine or a derivative thereof.

European patent EP 1827136 B1 describes a composition for oral administration having a beneficial health effect on the cardiovascular apparatus, comprising one or more polycosanols, red yeast and an antioxidant chosen from astaxanthin and folic acid.

European patent EP 1983989 B1 describes a synergistic combination of berberine with plant sterols or stanols (phytosterols or phytostanols) for lowering the level of lipids in the blood.

European patent EP 2007429 B1 describes a composition for oral administration with a beneficial health effect on the cardiovascular apparatus, comprising one or more polycosanols, berberine, red yeast and an antioxidant (folic acid or astaxanthin). The food supplement Armolipid Plus® is a commercial product produced on the basis of the teaching of said patent.

European patent EP 2205253 B1 describes a composition for preventing and treating inflammatory disorders, comprising (i) a probiotic, (ii) an omega-3 fatty acid, (iii) vitamin E, (iv) ubiquinone (also known as coenzyme Q), and (v) berberine.

European patent application EP 2626077 A2 describes a food supplement for improving bone and articular integrity, comprising a combination of at least two components chosen from various natural products, among which are mentioned *Abelmoschus* (gumbo or ocra), acacia extract, African devil's claw, bovine Arthred, porcine Arthred, astragalus, berberine, *Actaea racemosa,* chicken collagen, curcumin, devil's claw, dehydroepiandrosterone (DHEA), dioscorea, linseed, fructo-oligosaccharides (FOS), *Fructus ligustri,* genistin, glabridin, glucosamine, green tea, polyphenols from green tea, hesperidin, hyaluronic acid, inulin, ipriflavone, linoleic acid, myelin basic protein (MBP), MCHA, oleanolic acid, oleuropein, olive oil, osteosine, parthenolide, Perilla oil, phloridzin, pueraria root, pomegranate, quercetin, red rice, resveratrol, rho-iso-alpha acids (RIAA), rosemary, rutin, tetrahydro-iso-alpha acids (THIAA), vitamin K2 and withania.

European patent EP 1617836 B1 describes the use of oleuropein for preventing the reduction of bone tissue with ageing (osteopaenia).

European patent EP 1098573 B1 describes a method for extracting oleuropein, a substance described in numerous scientific studies since 1993 for its antiviral, antifungal, antibacterial, antioxidant and antiinflammatory properties.

### SUMMARY OF THE INVENTION

The Applicant has found, surprisingly, that a composition comprising a mixture of (i) berberine, or an extract or a product containing berberine, (ii) monacolin K, or an extract or a product containing monacolins, and (iii) oleuropein, or an extract or a product containing oleuropein, is particularly effective in reducing the level of LDL cholesterol in human blood.

The Applicant has also observed that the above-mentioned composition was also effective in reducing the total cholesterol and triglycerides in human blood, without significantly altering the amount of HDL cholesterol and the values for the serum enzymes (AST, ALT, CPK) and of urea.

The Applicant has thus observed that oleuropein promotes an entirely unexpected additive and synergistic effect on the reduction of cholesterol and triglycerides favoured by the administration of berberine and monacolin.

In a first aspect, the present invention thus relates to a composition comprising a mixture of (i) berberine, or an extract or a product containing berberine, (ii) monacolin K, or an extract or a product containing monacolins, and (iii) oleuropein, or an extract or a product containing oleuropein.

Preferably, the composition of the present invention also comprises an antioxidant, in particular ubidecarenone.

In a second aspect, the present invention relates to the use of a composition comprising a mixture of (i) berberine, or an extract or a product containing berberine, (ii) monacolin K, or an extract or a product containing monacolins, and (iii) oleuropein, or an extract or a product containing oleuropein, for preventing and/or treating hypercholesterolaemia or hypertriglyceridaemia.

In a third aspect, the present invention relates to the use of a composition comprising a mixture of (i) berberine, or an extract or a product containing berberine, (ii) monacolin K, or an extract or a product containing monacolins, and (iii) oleuropein, or an extract or a product containing oleuropein, for preparing a medicament or a food supplement which is useful in the prevention and/or treatment of hypercholesterolaemia or hypertriglyceridaemia.

### DETAILED DESCRIPTION OF THE INVENTION

The composition of the present invention comprises a mixture of
(i) berberine, or an extract or a product containing berberine,
(ii) monacolin K, or an extract or a product containing monacolins, and
(iii) oleuropein, or an extract or a product containing oleuropein.

Berberine is a quaternary isoquinoline alkaloid having the structural formula (I) below:

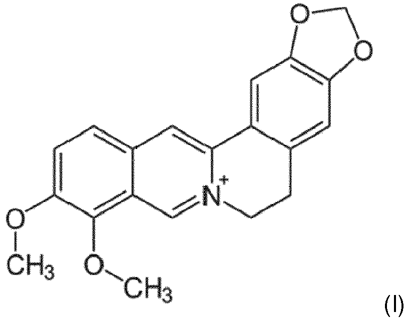

Normally, berberine consists of the chloride (Cl⁻) of the compound of formula (I), but other organic or mineral anions may be used to neutralize the charge of the quaternary nitrogen, for instance hydroxide ions (OH⁻), bromide ions (Br⁻), acetate ions (CH₃COO⁻), and the like.

Berberine is found in various plants of the *Berberidacea* family, such as *Berberis vulgaris, Berberis aquifolium* and *Berberis aristata,* and in other plant species, such as *Coptis chinensis, Hydrastis canadensis* and *Phellodendron amurense.*

Depending on the species and/or variety, berberine is predominantly localized in the roots, in the rhizomes, in the bark or in the stalks. Its content is often high: for example, *Coptis chinensis* rhizomes contain about 6% by weight and *Phellodendron amurense* bark contains about 4%.

Berberine and natural extracts thereof containing it are commercially available, for example, from the company Nutra Green Biotechnology Co., Ltd., United Kingdom.

The composition of the present invention comprises an amount of berberine of between 40% and 60% by weight, preferably between 45% and 55% by weight, relative to the total weight of the composition.

Advantageously, the composition of the present invention comprises about 50% by weight of berberine, relative to the total weight of the composition.

When the composition of the present invention uses natural extracts or products containing berberine, for instance extracts of *Berberis aristata,* the amount of natural extract or product containing berberine (for example, extracted from *Berberis aristata*) used in the composition will be an amount so as to give an amount of berberine of between 40% and 60% by weight, preferably between 45% and 55% by weight, relative to the total weight of the composition.

Monacolins are a group of molecules produced by fermentation of rice with the yeast *Monascus purpureus.* The fermentation of *Monascus purpureus* has been known in Chinese plant therapy and nutrition for centuries. The yeast is added to rice (*Oryza sativa*) to enable its fermentation and the product obtained, known as red rice, is used in the food sector.

Monacolins have a structure similar to that of statins. Monacolin K has the same structure (II) as lovastatin, reported hereinbelow.

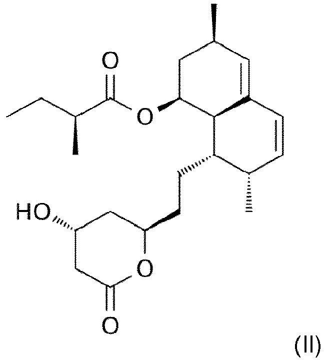

Fermented red rice may be obtained via known methods, for example as described in European patent EP 1044009 B1. Fermented red rice containing monacolin is a product that is commercially available, for example from the company Nutra Green Biotechnology Co., Ltd., United Kingdom.

The composition of the present invention comprises an amount of monacolin K of between 0.1% and 5% by weight, preferably between 0.5% and 2% by weight, relative to the total weight of the composition.

Advantageously, the composition of the present invention comprises about 1% by weight of monacolin K, relative to the total weight of the composition.

When the composition of the present invention uses natural extracts or products containing monacolins, for instance fermented red rice, the amount of extract or product containing monacolins (for example red rice) used in the composition will be an amount so as to give an amount of monacolin K of between 0.1 % and 5% by weight, preferably between 0.5% and 2% by weight, relative to the total weight of the composition.

Oleuropein is a glucoside of the ester of hydroxytyrosol with elenolic acid having the structural formula (III) below:

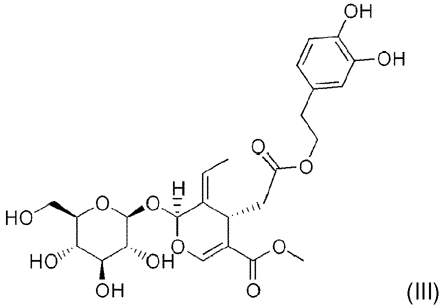

Oleuropein is the molecule that is by far the most abundant among the phenolic compounds present in the olive tree (*Olea Europea*) and in particular concentration in its leaves.

Oleuropein may be extracted from olives using the method described in European patent EP 1098573 B1 or from olive leaves using the method described in American patents US 6676980 and US 5714150. Olive leaf extracts containing oleuropein are commercially available, for example from the company Nutra Green Biotechnology Co., Ltd., United Kingdom.

The composition of the present invention comprises an amount of oleuropein of between 0.5% and 5% by weight, preferably between 1% and 3% by weight, relative to the total weight of the composition.

Advantageously, the composition of the present invention comprises about 2% by weight of oleuropein, relative to the total weight of the composition.

When the composition of the present invention uses natural extracts or products containing oleuropein, for instance olive leaf extracts, the amount of natural extract or product containing oleuropein (for example olive leaf extracts) used in the composition will be an amount so as to give an amount of oleuropein of between 0.5% and 5% by weight, preferably between 1% and 3% by weight, relative to the total weight of the composition.

Preferably, the composition of the present invention also comprises an antioxidant, in particular ubidecarenone.

Ubidecarenone (also known as coenzyme Q10 or ubiquinone) is a molecule of the ubiquinone group having the formula (IV) below:

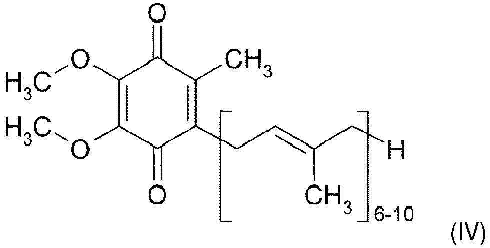

The composition of the present invention preferably comprises an amount of ubidecarenone of between 0.05% and 2% by weight, more preferably between 0.1 % and 1% by weight, relative to the total weight of the composition.

Advantageously, the composition of the present invention comprises about 0.2% by weight of ubidecarenone, relative to the total weight of the composition.

The composition of the present invention may be a medicament or a food supplement.

The form of the composition of the present invention may be represented by any form that is useful for oral administration, for instance solution, suspension, syrup, compress, granules, pellets, capsules, lozenges and pills.

The medicament of the present invention comprises the composition of the present invention and a pharmaceutically acceptable excipient.

The term "pharmaceutically acceptable excipient" is intended to mean, without particular limitations, any material that is suitable for preparing a pharmaceutical composition to be administered to a human being. Depending on the role played, the excipients are classified as (i) filling excipients, (ii) production excipients, (iii) storage excipients, and (iv) presentation excipients.

These materials, which are known in the art, are for example (i) diluents, absorbents, adsorbents, fillers and humectants (ii) lubricants, binders, glidants, plasticizers and viscosity modifiers, (iii) preserving agents, antimicrobial agents, antioxidants and chelating agents, and (iv) flavouring agents, sweeteners and colorants.

The food supplement of the present invention comprises the composition of the present invention and an edible excipient.

Advantageously, the food supplement of the present invention comprises at least one edible ingredient chosen from the group comprising carbohydrates, proteins, amino acids and derivatives, lipids, phospholipids, vitamins and mineral salts.

The food supplement of the present invention may contain other conventional food additives for improving its appearance, appeal and storage, for instance colorants, preserving agents, antioxidants, acidity regulators, thickeners, stabilizers, emulsifiers, flavour enhancers, flavourings, humectants and sweeteners.

Preferably, the medicament or food supplement according to the present invention is prepared in the form of capsules or tablets having a total weight of between 200 and 2000 mg, preferably between 500 and 1500 mg. Advantageously, the medicament or food supplement according to the present invention is a capsule or tablet having a total weight of about 1000 mg.

The preparation of the medicament and/or supplement of the present invention may be performed by applying techniques that are well known to any expert in the chemical-pharmaceutical field, which comprise, by way of example, for the preparation of a tablet, the sieving and weighing-out of the ingredients of the composition, dry or wet granulation of the mixture of all the ingredients or of part thereof, drying to remove any traces of humidity, harmful to the storage of the product, the preparation of the final composition and distribution in the appropriate tableting machine for the preparation of the tablet, dusting of the resulting tablets, and the optional final phase of film-coating.

When the medicament and/or supplement of the present invention is packaged in capsules, the final composition is introduced in the appropriate amount in an encapsulating machine in which the capsules are filled, closed and sealed.

When the medicament and/or supplement of the present invention is packaged in single-dose sachets, the final composition is introduced in the appropriate amount into sachets, which are subsequently sealed.

Pharmaceutical forms thus obtained, tablets, capsules or sachets, are then packaged and stored until the time of their distribution and sale.

The present invention is now illustrated with reference to the nonlimiting examples that follow.

### Example - Experimental clinical study

The experimental clinical study was performed in randomized double-blind mode using three groups of volunteers to whom were administered the placebo product (PL), the product of the invention (BL), in the form of 1 g capsules comprising the ingredients of Table 1, and the commercial product Armolipid Plus® (AM), a food supplement in capsule form containing berberine extracts (550 mg, equivalent to 500 mg of berberine), fermented red rice (200 mg, equivalent to 3 mg of monacolin K), policosanol (10 mg), folic acid (0.2 mg), coenzyme Q10 (2 mg) and extract of microalgae (20 mg, equivalent to 0.5 mg of astaxanthin) described in Example 1 of European patent EP 2007429 B1.

**TABLE 1**

| Ingredient | Amount (mg) |
|---|---|
| Dry extract of *Berberis aristata,* berberine titre (85%) | 588.23 (500.00) |
| Dry extract of fermented red rice, monacolin K titre (5%) | 200.00 (10.00) |
| Dry extract of *Olea europea,* oleuropein titre (35%) | 50.00 (17.50) |
| Ubidecarenone (Coenzyme Q10) | 2.00 |
| Croscarmellose sodium | 5.00 |
| Anhydrous biphasic calcium phosphate | 100.00 |
| Precipitated/colloidal silica | 5.00 |
| Plant magnesium stearate | 5.00 |
| Microcrystalline cellulose | 44.75 |

The volunteers used in the clinical study were selected on the basis of the following criteria:
1. Men or women between 18 and 75 years old
2. Values at the first visit (V1)
   - LDL > 115 mg/dl
   - TC > 200 mg/dl
   - Tg < 250 mg/dl
3. Values at the second visit (V2)
   - LDL > 115-180 mg/dl
   - TC > 200-260 mg/dl
   - Tg < 250 mg/dl

During the visits, besides the above-mentioned LDL, TC and triglyceride (Tg) values, the HDL values, the serum enzyme (AST, ALT, CPK) values and the urea values were also determined.

Between the first and the second visit, the volunteers were subjected to a controlled diet for four weeks, adhering to the following general rules:
1. Consuming three meals per day (breakfast, lunch and supper) with two snacks between the meals
2. Avoiding abundant meals and the sensation of weight on the stomach
3. Not eating in the same meal:
   - Bread and pasta
   - Rice, bread and potato, pasta
   - Rice and potato
4. Favouring pasta/rice for lunch and meat/fish for supper
5. Starting the meal, if possible, with raw vegetables, seasoned with oil, lemon and vinegar
6. Not going to bed immediately after a meal.

After the second visit, the volunteers were divided randomly into three groups of 50 persons each. Each person of each group was given 35 capsules of the respective treatment PL, BL or AM.

The volunteers continued the controlled diet, taking one capsule per day of the product given to them for four weeks. The product was taken in the evening during or immediately after supper with a glass of water.

At the end of the treatment, the volunteers were subjected to a final visit (V3) to determine all the TC, LDL, HDL, Tg, AST, ALT, CPK and urea values obtained.

Table 2 below summarizes the results obtained, expressed as the average for each group, and as the difference between the groups which took the product of the invention (BL) or the comparative product (AM), and the group which took the placebo (PL). The differences between V3 and V1 were calculated and compared by means of a covariance or variance analysis (ANCOVA or ANOVA) to reveal the significant differences between the groups PL, AM and BL.

**TABLE 2**

| **TC** | V1 | V3 | Δ | Δ% |
|---|---|---|---|---|
| PL | 235.6 | 238.0 | +2.4 | +1.2 |
| AM | 234.6 | 204.9 | -29.7 | -12.6 |
| BL | 237.0 | 191.2 | -45.9 | -19.1 |
| | | | | |

| **LDL** | V1 | V3 | Δ | Δ% |
|---|---|---|---|---|
| PL | 143.6 | 149.3 | +5.7 | +4.2 |
| AM | 147.5 | 120.4 | -27.0 | -18.3 |
| BL | 146.4 | 107.3 | -39.1 | -26.3 |
| | | | | |

| **HDL** | V1 | V3 | Δ | Δ% |
|---|---|---|---|---|
| PL | 70.0 | 68.1 | -1.9 | -2.0 |
| AM | 65.1 | 63.4 | -1.7 | -2.4 |
| BL | 66.8 | 62.2 | -4.6 | -6.5 |
| | | | | |

| **Tg** | V1 | V3 | Δ | Δ% |
|---|---|---|---|---|
| PL | 110.5 | 103.5 | -7.0 | -0.1 |
| AM | 110.8 | 105.7 | -5.1 | -1.4 |
| BL | 118.9 | 108.5 | -10.4 | -4.4 |
| | | | | |

| **AST** | V1 | V3 | Δ | Δ% |
|---|---|---|---|---|
| PL | 22.8 | 22.2 | -0.6 | -0.7 |
| AM | 23.3 | 23.6 | +0.3 | +6.7 |
| BL | 21.6 | 22.6 | +1.0 | +11.5 |
| | | | | |

| **ALT** | V1 | V3 | Δ | Δ% |
|---|---|---|---|---|
| PL | 22.5 | 21.0 | -1.5 | -3.4 |
| AM | 22.2 | 23.5 | +1.3 | +9.4 |
| BL | 22.3 | 24.3 | +2.0 | +14.8 |
| | | | | |

| **CPK** | V1 | V3 | Δ | Δ% |
|---|---|---|---|---|
| PL | 109.2 | 115.9 | 6.7 | 17.9 |
| AM | 114.8 | 124.7 | 9.9 | 12.1 |
| BL | 95.6 | 97.6 | 2.0 | 6.2 |
| | | | | |

| **UREA** | V1 | V3 | Δ | Δ% |
|---|---|---|---|---|
| PL | 35.7 | 35.6 | -0.1 | 0.8 |
| AM | 35.8 | 35.1 | -0.7 | -0.1 |
| BL | 35.3 | 36.2 | 0.9 | 4.9 |

| | | | | |
|---|---|---|---|---|
| V1: Values obtained at the first visit V3: Values obtained at the end of the treatment Δ : Absolute difference between V3 and V1 Δ %: Percentage difference between V3 and V1 | | | | |

Table 3 below summarizes the values P for the statistical analysis performed for α = 0.05 and using the software SAS version 9.2 (SAS Institute Inc., North Carolina, USA). All the values were statistically significant.

**TABLE 3**

| | PL | AM | BL |
|---|---|---|---|
| TC | 0.319 | 0.000 | 0.000 |
| LDL | 0.026 | 0.000 | 0.000 |
| HDL | 0.232 | 0.148 | 0.000 |
| Tg | 0.078 | 0.180 | 0.066 |
| AST | 0.796 | 0.653 | 0.404 |
| ALT | 0.494 | 0.276 | 0.157 |
| CPK | 0.463 | 0.267 | 0.807 |
| Urea | 0.710 | 0.327 | 0.471 |

The data in Table 2 demonstrated, surprisingly, that the composition of the present invention is capable of significantly reducing the values for total cholesterol (TC) and for LDL cholesterol (LDL) relative to the placebo and to the comparative product. It is worthwhile noting that a reduction was also observed in the triglyceride (Tg) values.

No significant variations were observed for HDL, serum enzymes and urea.

The product of the invention thus gave a more effective overall result and with lesser contraindications when compared with the comparative product.

The addition of oleuropein thus induces a surprising and unexpected additive and synergistic effect on the effect of reducing cholesterol and triglycerides that is intrinsic to berberine and monacolins, bringing the haematic cholesterol and triglyceride values to virtually normal levels.

The composition of the present invention is thus advantageously suitable for use in preventing and/or treating hypercholesterolaemia or hypertrigliceridaemia, either as is or in the preparation of a medicament or a food supplement that is useful in the prevention and/or treatment of hypercholesterolaemia or hypertrigliceridaemia.

## Claims

1. A composition comprising a mixture of (i) berberine, or an extract or product comprising berberine, (ii) monacolin K, or an extract or product comprising monacolins, and (iii) oleuropein, or an extract or product comprising oleuropein.

2. The composition according to claim 1, **characterized in that** said composition comprises an amount of berberine ranging from 40% to 60% by weight, preferably from 45% to 55% by weight, with respect to the total weight of said composition.

3. The composition according to claim 1, **characterized in that** said composition comprises an amount of monacolin K ranging from 0.1 % to 5% by weight, preferably from 0.5% to 2% by weight, with respect to the total weight of said composition.

4. The composition according to claim 1, **characterized in that** said composition comprises an amount of oleuropein ranging from 0.5% to 5% by weight, preferably from 1% to 3% by weight, with respect to the total weight of said composition.

5. The composition according to claim 1, **characterized in that** said composition further comprises an antioxidant.

6. The composition according to claim 1, **characterized in that** said antioxidant is ubidecarenone.

7. The composition according to claim 6, **characterized in that** said composition comprises an amount of ubidecarenone ranging from 0.05% to 2% by weight, preferably from 0.1% to 1% by weight, with respect to the total weight of said composition.

8. The composition according to claim 1, **characterized in that** said composition is a medicament.

9. The composition according to claim 8, **characterized in that** said composition comprises at least one pharmaceutically acceptable excipient.

10. The composition according to claim 9, **characterized in that** said pharmaceutically acceptable excipient is selected from the group comprising (i) diluents, absorbents, adsorbents, fillers and wetting agents, (ii) lubricants, binders, glidants, plasticizers and viscosity modifiers, (iii) preservatives, antimicrobials, antioxidants and chelating agents, and (iv) flavoring agents, sweeteners and dyes.

11. The composition according to claim 1, **characterized in that** said composition is a food supplement.

12. The composition according to claim 11, **characterized in that** said composition comprises at least one edible excipient.

13. The composition according to claim 12, **characterized in that** said edible excipient is selected from the group comprising carbohydrates, proteins, amino acids and derivatives, lipids, phospholipids, vitamins and minerals.

14. The composition according to claim 13, **characterized in that** said composition further comprises a food additive selected from the group comprising coloring agents, preservatives, antioxidants, acidity regulators, thickeners, stabilizers, emulsifiers, flavor enhancers, flavorings, humectants and sweeteners.

15. Use of a composition comprising a mixture of (i) berberine, or an extract or product comprising berberine, (ii) monacolin K, or an extract or product comprising monacolins, and (iii) oleuropein, or an extract or product comprising oleuropein in the prevention and/or treatment of hypercholesterolemia or hypertriglyceridemia.

16. Use of a composition comprising a mixture of (i) berberine, or an extract or product comprising berberine, (ii) monacolin K, or an extract or product comprising monacolins, and (iii) oleuropein, or an extract or product comprising oleuropein for the manufacture of a medicament or a food supplement useful for the prevention and/or treatment of hypercholesterolemia or hypertriglyceridemia.
